# EUROPEAN PATENT APPLICATION

(11) **EP 3 701 870 A2**
(43) Date of publication of application: **02.09.2020**
(21) Application number: 20160111.9
(22) Date of filing: 04.10.2017
(51) Int. Cl.: A61B 6/00, A61B 6/10, G06F 3/00, A61B 6/08, A61B 6/06, A61B 6/04, A61B 6/12

(54) **METHOD OF ADJUSTING SETTINGS OF A RADIATION IMAGE RECORDING SYSTEM**

(30) Priority: 14.10.2016 EP 16193901
(62) Divisional of application: 17777591.3
(71) Applicant: AGFA NV, 2640 Mortsel (BE)
(72) Inventor: BEHIELS, Gert, 2640 Mortsel (BE)
(74) Representative: Verbrugghe, Anne Marie L.

(57) **Abstract**

Spatial change of a body part of an operator of a radiation image recording system is tracked and measured and setting(s) of the radiation image recording system are adjusted by an amount proportional or equal to the measured spatial change.

## Description

### FIELD OF THE INVENTION

The present invention relates to a method for adjusting settings of radiation image recording system, e.g. an x-ray source, an x-ray collimator, a supporting table or setting(s) of an application, setting(s) of a workflow etc.

### BACKGROUND OF THE INVENTION

In traditional analogue radiography, used e.g. in medical applications, imaging is performed by means of a light sensitive photographic film in combination with a phosphor layer which converts the incident X-rays to visible light. The light emitted by
the phosphor is captured by the film which is developed to obtain a image on the film. Different sizes of assemblies which may be conceived as cassettes or as film packages are used in daily practice. The drawback of film based systems is that they require that the photographic film has to be chemically processed, leading to chemical waste products and loss of time.

More recently, digital X-ray systems, now known as computed radiography (CR) systems are used. These systems use a stimulable phosphor which is exposed to a radiation image. The stimulable phosphor stores the radiation image at exposure. Next the stored image is read out by scanning the phosphor by means of stimulating radiation. Upon stimulation image-wise stored energy is emitted as light. The emitted light is then detected and converted into an electronic image which is digitized.

Digital radiography (DR) is another form of X-ray imaging, where digital X-ray sensors are used instead of traditional photographic film or cassette based CR systems. Advantages include time efficiency through bypassing chemical processing (compared to traditional film based systems) and through immediate read-out of the image data from the sensor (compared to cassette based CR systems where the read-out of the detector is done by means of a dedicated digitizer system).

In all these systems the equipment for generating a radiation image of an object or a patient comprises an x-ray source, an x-ray collimator which collimates the x-rays emitted by the source of radiation into a cone of radiation that irradiates a region of interest on the patient or the object, a supporter that supports the source of radiation including the collimator and that enables positioning of the radiation source relative to the patient or the object to be irradiated, a support table for supporting the patient or the object and containing the imaging capturing means, means for controlling operation of the x-ray source, the collimator, the patient or object support and means for operating these items under control of the controlling means.

Conventionally the X-ray tube is arranged in a housing which also comprises a collimator to collimate x-rays generated by the x-ray source onto a region of interest.

The housing comprising the x-ray source and the collimator, can be moved by means of a positioning system in two perpendicular directions (x-direction and y-direction) and additionally allows for vertical movement in a z-direction and rotation around at least two axis.

The collimator consists of a number of x-ray opaque collimator blades that can be moved relative to each other so as to enlarge or decrease an area through which x-rays emitted by the x-ray source can pass so as to delimit irradiation to a region of interest on the patient or object.

Different types of collimators exist.

Collimators can be of a symmetric type so that collimator blades are always moved together so that the shape of the aperture formed in between the collimator blades is not changed only the dimensions of the opening through which the x-rays are allowed to pass towards the patient or object may vary.

Another type of collimator is the asymmetric type. In such a collimator the collimator blades can be moved independently from each other which amounts to a shift of the irradiated field of interest relative to the patient. This shift can be achieved by only moving opposite blades of the collimator.

The movement of the collimator blades may be either fully automatic, semi automatic or manual. In the manual embodiment X-ray apparatus includes mechanical actuated means to manually effect and control the motion of the collimator blades.

In the semi automatic embodiments the user controls the operation of motors or other means by operating e.g. buttons on the user console so as to move the collimator blades.

In the fully automatic embodiment the blades are responsive to collimator controller that issues control commands upon receiving collimator control information without requiring user interaction.

However, in all the described situations it is cumbersome to exactly know the position of the area of interest and to manipulate the collimator so that the x-rays emitted by the source of radiation are directed towards the region of interest.

Especially in the case mobile x-ray irradiation devices are used and detector nor patient are in a fixed position, it is cumbersome to accurately define the area of interest and set out the collimator blades accordingly.

There is thus a need for improved collimator setting procedure. This problem is dealt with in the present invention.

Although the problem has been identified with regard to the settings of the collimator blades, for all other settable components of the x-ray recording system a similar problem exists.

Optimal positioning of the system components is cumbersome and often requires more than one attempt to obtain the envisaged position.
This takes time to match the position intended by the operator to the actual position. The fact that this takes time and requires repeated actions may be very annoying for the patient as well as for the operator.

Additionally if the direction of the irradiation needs to be angulated, precise positioning involves two operations where one operation affects the adjustment of the other. It would be more easy to indicate the region of interest directly and adjust the settings of all components accordingly.

Furthermore, it is highly desirable that the operator should not have to touch the device so as to minimize the chance of infections in a medical environment.

Prior art publications exist in which manipulation of devices in a medical environment are performed without the operator having to touch the device nor the patient.

US 2015/0359497 discloses an X-ray diagnostic apparatus which includes a display, a holding device, a bed device and a gesture detection device and processing circuitry. The holding device includes an X-ray irradiator and an X-ray detector and a supporter that supports the X-ray irradiator and the X-ray detector.
A gesture detection device recognizes the gestures of a person. The processing circuitry identifies the state of the X-ray diagnostic apparatus based on at least one of display, X-ray irradiator, X-ray detector, holding device and bed device and determines an operation detail on a combination of the identified state and the recognized gesture and operates at least one of the display, the holding device, the bed device a speaker and a room light according to the determined operation detail.

According to this method correcting or changing a setting requires the procedure to start all over again. This includes initialisation of the detection procedure, setting and closing of the detection procedure. This is again cumbersome and time-consuming.

It is thus an aspect of the present invention to improve the procedure for adjusting setting(s) of radiation image recording system.

### SUMMARY OF THE INVENTION

The above-mentioned aspects are solved by a method as set out in claim 1.

Specific features for preferred embodiments of the invention are set out in the dependent claims.

Further advantages and embodiments of the present invention will become apparent from the following description.

The present invention describes a method of adjusting the settings of a radiation image recording system. These settings may be settings of a component of the recording system or of an application performed on such a system or a workflow performed on such a system.
A radiation image may be taken from an object, a human patient or an animal. Whenever hereinafter reference is made to a patient it is to be understood that patient can be replaced by object or animal.

Radiation images may be generated by applying one of different kinds of radiation. Among these different kinds of radiation are x-rays, ultrasound, Magnetic Resonance Imaging (MRI), Optical Coherence Tomography (OCT) etc. The present invention is applicable to radiation image recording systems using one of these different types of radiation.

Unless otherwise specified, x-rays may be changed in to another kind of radiation in the description below.

The invention is likewise applicable to radiation image recording systems on which different kinds of radiation image recording methods are performed.

Among these types of recording methods the most conventional is projection radiography wherein x-rays emitted by a source of radiation are projected onto a 2 dimensional area on the patient to be imaged.

Another type of an x-ray imaging method is computed tomography (CT) wherein a plurality of x-ray images are taken from different angles to produce cross-sectional slice images.
A reconstructed image is computed by applying a reconstruction algorithm to the slice images.

A specific type of tomography is cone beam computed tomography wherein the X-rays are divergent, forming a cone.

Still other radiation imaging techniques exist such as fluoroscopy which generates real time images of the body.

The invention is likewise applicable to radiation image recording systems on which different applications are performed such as full leg, full spine imaging.

Radiation image recording systems may be in a fixed position in an x-ray room such as in the case of an x-ray bucky device or an x-ray wall stand.

Alternatively these systems may be mobile so that they can be moved to the location where the patient resides, e.g. in a hospital bed in an intensive care unit. An example of such a system is Agfa HealthCare's DX-D 100 mobile X-ray unit.

The present invention is applicable to either of these types.

Suchlike systems comprise several components that require adjustments of certain settings. The adjustment of the components of these systems can be done completely or semi-motorized or completely operated by manual force.

A first component is the radiation source, e.g. an X-ray source that is movable in order to be positioned above a region of interest to be irradiated or positioned angulated and directed to the region of interested to be irradiated. The setting of the x-ray source itself or of X-ray source supporting means supporting the X-ray source and / or moving it from one location to another needs to be performed.

In order to collimate the X-rays emitted by the x-ray source, a collimator device is provided in front of the x-ray emitting face of the x-ray source. The collimator consists of a number of collimator blades out of x-ray blocking material that are arranged so as to define a diaphragm through which x-rays are directed towards the patient. The collimator blades can be moved so as to enlarge or decrease the aperture area.

Still another component is the supporting table on which the object (patient) to be irradiated is positioned.

Still other components may be envisaged in the context of the present invention.

In case of a specific application or workflow such as a full leg / full spine image recording certain settings are to be performed. For example, start and stop position of the recording area need to be set.

According to the present invention the settings of components or the settings of an application or steps in a workflow of the x-ray imaging system is controlled by tracking the movement of a body part of an operator.

The operator performs a movement of a body part (operator's body part) such as a hand during a certain amount of time, starting at a start tracking moment and continuing until a stop tracking moment.
The movement can be a non-contact movement, i.e. without contacting the patient or any of the modality components. The movement can also be a contact movement, where contact can be a physical contact where the operator touches any of the modality components with a defined gesture or a virtual contact where the operator touches the boundary of the light cone of the illuminated area of the collimation field, for example.

Depending on the type of action that is controlled by the tracked body part movement this movement is performed in the neighbourhood of the patient or can be performed elsewhere. For example when hand movements are tracked to delineate a region of interest, this is preferably performed close to the actual region of interest on the patient. For other actions such as movement of the X-ray source transporting means this is less important.

The body part can e.g. be a hand or both hands (e.g. defining an area by means of two hands the fingers of which delineate the area). However, other body parts may be envisaged such a foot (both feet), the operator's head etc. Various embodiments may be thought of.

The movement of the body part is tracked from a start tracking moment until a stop tracking moment.
Start and stop of the tracking can be indicated in several ways.

In a first example start and stop of the tracking is controlled by the position of the operator or of a body part of the operator.
For example, when the operator stands in a pre-defined location, e.g. a marked location, in the radiology room and this position is detected, tracking of movement of one of his hands is performed.
When the operator is in a second predefined location or moves away from the first predefined area this moment is identified as tracking end moment.
In a specific embodiment the first and second location can even be the same location, however in this case the operator has to move away from this location during tracking and to move again to the location when he wants to stop the tracking and the measurement.

In another example start and stop tracking can be indicated by means of a gesture that is registered and recognized. For example, the operator can perform a "thumb up" gesture to start the tracking and a stop or for example "thumb down" gesture to stop tracking.

In still alternative embodiments start and stop tracking can be controlled by means of an audio signal or by means of a voice command.

Still alternative embodiments and alternative combinations of start and stop actions or signals are possible.

In order to avoid detection and tracking of body parts of other persons present in the radiology room, the operator is first identified to the means which track the movement of the body part.

Operator identification can be performed in different ways.

In one example the operator is identified by face recognition or by registering and checking biometric data of the operator.

In another embodiment the operator is identified by his position, e.g. if a person stands in a certain position in the radiology room, this person is identified as being the operator.

The identification of the operator as well as the tracking of the body part movement can be performed by recording this movement by means of at least one camera.
Multiple cameras or a 3D camera can be used to get 3D depth information on the instantaneous location of the body part so that tracking of the location of that body art can be performed.

Face recognition can be implemented by determining a set of predefined features and computing a similarity measure between these features. A collection of features defining the operators is stored on the workstation or any other storage device. For each person identified and tracked in the camera image, a similarity measure is computed. The person with the highest similarity measure with any of the operators stored on a workstation is defined as the person which can operate the modality. Optionally other constraints on the similarity measures can be defined to restrict operation of the modality.

Finally the change of movement of the body part in between start and stop of the tracking is measured and at least one component of the radiation image recording system or a setting or step of an application or workflow is changed by an amount equal to or proportional to the tracked and measured location change of the body part.

In cases where the direction of the change of movement is relevant, for example in case the settings of component of the recording device which is to be positioned in a certain location is changed, the amount of movement preferably includes the direction of the change of movement.

For example when the position of the x-ray source relative to the patient is set according to the present invention in accordance with a tracked and measured change of position of a hand of the operator, then the direction in which the hand has been moved is also the direction in which the x-ray source will be moved. The distance or amount by which the x-ray source will be moved will then be identical or proportional to the tracked and measured amount of movement of the body part.

The proportionality factor is preferably determined in advance and occasionally stored by controlling means which are coupled to the device that tracks the movement of the body part so as to perform the computation of the required setting on the basis of the measured spatial change of the body part.

The amount of spatial change can be displayed on the operator's workstation.

The present invention provides that the input of the region from which an X-ray image must be taken is more accurate and more efficient. Up till now, the radiographer had to operate the X-ray tube and the collimator to select the region. This operation involves touching the equipment. Because of the specific nature of the input, the X-ray tube may not have been perfectly centered to the region, which leads to slightly wider collimated areas and off center exposures.

This invention solves this problem by letting the radiographer indicate directly on the patient which region he would like to image. Afterwards the modality positions and the collimator is automatically set to image this region. When the operator changes the collimation area, the collimator settings follow the changes made by the operator.

Manipulation of buttons or controls is avoided, the operator performs gestures without using any object. This procedure is far more intuitive.

As a cross check, the modality may project visible light from the collimator onto the selected region of interest. The radiographer can refine the region, either with additional gestures or with standard input as currently is implemented by all modalities.

### DETAILED DESCRIPTION OF THE INVENTION

The invention will now be described in detail for the particular situation in which the position of the radiation source is set and in which a region of interest is determined by means of an x-ray collimator and occasionally changed by applying the method of the present invention.

When an X-ray image of a body part of a patient is to be taken, the patient is positioned with the aid of an operator in a suitable position for x-ray image recording. Depending on the type of examination the patient is positioned on a so-called wall stand in a vertical position or alternatively he is positioned on a supporting table in a horizontal position.

An intelligent patient analysis is then performed. First the patient is identified. Patient data may be entered in a workstation coupled to the x-ray recording device or they may be retrieved from a radiology information system (RIS).

Next the patient's weight and length are measured and the patient's body mass index is calculated. From this body mass index the body type of the patient can be derived. In accordance with the patient's body type, the radiation dose adequate for image recording is derived. In addition, the patient's thickness of the specified body part can be derived from the depth measurements from the camera.

The patient's weight can be measured with a sensor in front of the wallstand or in the support table. The patient's height can be derived from the depth measurements. The height measurements can be done directly or indirectly based on a skeletonization of the depth measurements and after identification of the patient.

In one embodiment patient data (such as name, photo of patient, length, weight, body mass index) are projected onto the wall of the x-ray recording room and/or on an additional monitor or display device attached to the modality or detachable from the modality so that the operator as well as the patient himself can verify the data. In this way errors can be avoided.

Next, the settings for the x-ray source are determined and set: if the body part of the patient is known and is successfully tracked with the camera, the position of the this body part is mapped from the camera's coordinate system to the coordinate system of the modality and the modality is positioned as best as possible to a position which is optimal for the requested acquisition protocol. In addition the size and position of the collimated area is adjusted based on the size measurements and position of the patient. Additionally, dose acquisition parameters such as kV and mAs can be adapted to fit the patient's fysiology as good as possible. Hereby the thickness of the patient's body part, patient's body type and tissue type of the body part to be irradiated can be taken into account.

Then the position of the x-ray source including the collimator is to be set or fine-tuned so that x-rays emitted by the source of radiation irradiate the region of interest.

In this specific embodiment of the invention, the position of the source of radiation relative to the patient as well as the setting of the collimator blades is controlled by means of hand gestures (possibly non-contact: no contact with the patient, nor the recording device) of the operator and tracking of the change of the location of these hands. It is also possible to position the source of radiation and collimated area with standard input as currently is implemented.

In order to avoid mistakes when tracking the hand movements of the operator, the operator is first to be identified so that only his hand movements and not these of another person that is present in the room (e.g. the patient) are tracked and used for setting of the location of the x-ray source and the collimator.

For that purpose in this specific embodiment a picture of the operator is taken by means of at least one of the cameras that is provided in the x-ray room. One camera which has a field of view containing the operator and patient is sufficient but also multiple cameras can be used. If the positioning of the cameras is known with respect to each other or with respect to the modality, the information of the multiple cameras can be merged to create a more detailed image or representation of the room.

Systems such as Microsoft Kinect and Intel RealSense identify and track persons within a video sequence. So once a person has been identified with face recognition, it is therefore possible to track this person with the associated identification label generated by the person tracking software. One could position a depth sensing camera or regular camera facing the entry of the modality room or positioned at a place where the patient and operator are guaranteed to pass. If a frame of such a camera is good enough for face recognition, the person identification from the face recognition is linked with person identification from the person tracking software.

The operator can be identified by face recognition and person tracking links. Alternatives are possible, for example on the basis of the location where the operator is standing a difference can be made between the operator and the patient (the patient being the person that lies on the supporting table or that stands on the wall stand, the operator being the person in the room that is not on the supporting table or on the wall stand). If even more persons are present in the room, the operator can be identified and tracked as the first person assisting the person on the supporting table or on the wall stand.

In a specific embodiment the generation of radiation is prevented when 2 or more persons are detected in a given area. Once a person is identified as being the operator, movements of a specified body part made by this person are taken into account for controlling the operation of components of the x-ray recording device. The movement of a body part will be measured and the amount of change of movement or an amount which is proportional to the measured amount will be used to control the positioning of the x-ray source as well as to adjust the collimator settings.

In order not to take into account body part movement, in this case hand movements, which were not intended to be used for controlling one of the above mentioned components, the movement tracking is only initiated once a tracking start indication is generated and detected.
Likewise the tracking is stopped once a stop tracking indication is given and detected.

This indication may have different forms. However one of the described embodiments the tracking start indication is a gesture in which each of the hands poses the thumb and index in an angle of approximately 90 degrees while closing the other fingers and the tracking stop indication is releasing the pose of this gesture.

In order to delineate a region of interest, the operator forms a rectangle with the fingers of both hands above the region of interest for x-ray imaging on the patient.

In another described embodiment, the tracking start indication is a gesture where both hands are positioned parallel as flat hands in either a vertical or horizontal plane and the tracking stop indication is the closure of one or both of the hands.

In order to adjust the width of the collimated area, the operator poses his hands parallel vertically. The distance between the start of this gestures defines the current width of the collimator. If the distance between the hands increases, the width of the collimator also increases proportionally. For example, the width is increased proportionally with the ratio between the width of the collimator and the width between the hands at the start indication moment. Another implementation would be to increase the width of the collimator identical to the increase of the distance between both parallel hands.

In order to adjust the height of the collimated area, a similar method is implemented for hands positioned parallel horizontally.

A depth camera provided in the x-ray room records the image of the hands and measures the area. This information is applied to the controller of the x-ray source and collimator and the collimator blades are adjusted so that they delineate an opening for x-rays emitted by the x-ray source to pass through which is proportional to the recorded area. The proportional factor can be the ratio between the area of the collimated area at the start tracking area and the area of the indicated area with the hands. Another possibility is that if the width or height of the indicated area increases or decreases with one cm, the corresponding width or height increases or decreases with one cm or a factor thereof.

From detection of the start tracking signal to detection of the stop tracing signal movements of the hands are recorded and measured by the depth camera and spatial changes of the hand positions (and consequentially of the area delineated by the hands) are applied to the controller of the x-ray source and the collimator. The collimator opening is adjusted in accordance with the detected and measured spatial change of the hand position.

Once the stop tracking signal is generated and detected, no tracking of the spatial change of the hand positions is performed anymore and no corresponding further changes are applied to the x-ray source and collimator.

Visual control by the operator can be obtained by displaying the hand movements on the display device of the operator's work station.

In order to have an additional check of the location of the region of interest on which x-rays will be projected, visible light is projected from the collimator position onto the patient, said visible light delimiting the region of interest.

As an alternative, the collimated area can be computed by taking into account the position of the 3D camera, the position of the X-ray source and the measured depth data. The estimated collimation area computed based on the known geometry, can be presented as an overlay on a (color) image from another camera or a (color) image from the visual camera in the same 3D camera system.

Once the position of the x-ray source and the collimation area are set to the satisfaction of the operator, a radiation image of the patient can be taken.

It is also possible to track the movement of the patient after the x-ray source and collimation area are set correctly. For example, depth measurement data of the collimation area can be taken after the final adjustment of the operator. This depth data can be registered with newly obtained depth measurements. If the registration differs from the initial position, the system can update the X-ray source and collimation area such that the original object of interest is imaged in the same manner. If this is not possible, a warning to the operator can be generated.

In addition, it is also possible that the modality checks if all acquisition parameters are set correctly. For example, based on the depth measurements and the location of the X-ray source, the system can compute if all active AEC chambers are covered by the patient. If this is not the case, the uncovered AEC chambers can be de-activated or a warning to the operator can be generated, e.g. by display.

In one embodiment a current position of items in an x-ray room is recorded and movement of parts in the radiology room, e.g. the x-ray source is controlled taking into account the recorded position so as to avoid collision with said items in the radiology room.

In another embodiment movement of a patient relative to a patient supporting device is tracked and settings, e.g. of an x-ray collimator are adapted taking into account said movement so that the collimation area retains the same relative location to the patient.

In still another embodiment the generation of radiation is prevented when 2 or more persons are detected in a given area. ■

## Claims

1. Method of adjusting settings of a radiation image recording system for recording a radiation image of a patient comprising the steps of
- performing a first action to start tracking of the position of at least one body part of an operator of said radiation recording system,
- performing a second action to stop tracking of the position of said body part performing a movement without contacting said recording system or said patient,
- measuring spatial change of said body part in between start and stop of the tracking,
- adjusting said settings by an amount proportional or equal to the measured spatial change, **characterised in that** said body part is recognized by performing the steps of registration of a reference body part with the measured depth image and by computing and interpreting a similarity measure.

2. Method according to claim 1 wherein said settings are settings of a component of the radiation image recording system.

3. Method according to claim 2 wherein said component is at least one of a radiation source, a radiation collimator, radiation source supporting means, a patient support table.

4. Method according to claim 1 wherein said settings pertain to one of setting steps of an application or one of setting steps of a workflow.

5. Method according to claim 1 wherein said first and / or said second action is one of a gesture, a positioning of a body part of said operator on a predetermined location, the generation of an audio signal, a voice command.

6. Method according to claim 1 wherein a 2 dimensional area is delimited by means of said body part and wherein said 2 dimensional area is detected and measured and measurement results are applied to a radiation collimator to delimit a collimation area.

7. Method according to claim 6 wherein said area is delimited by a square defined by means of the operator's hands and wherein zooming of the area delimited by these hands is performed to enlarge or decrease the 2 dimensional area and wherein the setting of the collimator to define a collimation area is adapted accordingly.

8. Method according to claim 1 wherein at least two gestures are performed in different directions and one of said gestures is implemented by means of parallel hands and wherein chance of the distance between the parallel hands is used to control increase or decrease of a setting in the direction of said change.

9. Method according to claim 1 wherein said tracing is performed by recording the position of said body part by means of at least one camera.

10. Method according to claim 9 wherein said camera is at least one depth camera.

11. Method according to claim 1 wherein a current position of items in a radiation image recording room is recorded and wherein movement of a component of the radiation image recording system is controlled taking into account the recorded position so as to avoid collision with said items in the radiology room.

12. Method according to claim 1 wherein movement of a patient relative to a patient supporting device is tracked and wherein settings of a component of the radiation image recording system are adapted taking into account said movement so that the component retains the same relative position to the patient.

13. Method according to claim 1 wherein the generation of radiation is prevented when 2 or more persons are detected in a given area.■
